# EUROPEAN PATENT SPECIFICATION

(11) **EP 3 609 874 B1**
(45) Date of publication and mention of the grant of the patent: **09.06.2021**
(21) Application number: 18720919.2
(22) Date of filing: 10.04.2018
(51) Int. Cl.: C07D 235/04

(54) **IONIC COMPOUNDS COMPRISING 1,3-DIALKYL-4,5,6,7-TETRAHYDRO-1H-BENZO[D]IMIDAZOL-3-IUM CATIONS FOR USE IN COATINGS AND ADHESIVES**
IONISCHE VERBINDUNGEN MIT 1,3-DIALKYL-4,5,6,7-TETRAHYDRO-1H-BENZO[D ]IMIDAZOL-3-IUM-KATIONEN ZUR VERWENDUNG IN BESCHICHTUNGEN UND KLEBSTOFFEN
COMPOSÉS IONIQUES COMPRENANT DES CATIONS 1,3-DIALKYL-4,5,6,7-TÉTRAHYDRO-1H-BENZO[D]IMIDAZOL-3-IUM POUR UTILISATION DANS DES REVÊTEMENTS ET DES ADHÉSIFS

(30) Priority: 11.04.2017 US 201762484211 P
(43) Date of publication of application: 19.02.2020
(73) Proprietor: Nitto Denko Corporation, Ibaraki-shi, Osaka 567-8680 (JP)
(72) Inventor: RACHWAL, Stanislaw, Oceanside, California 92057 (US); HU, Yufen, San Diego, California 92130 (US); ZHANG, Hongxi, Temecula, California 92592 (US); WANG, Peng, San Diego, California 92126 (US)
(74) Representative: Potter Clarkson
(86) International application number: PCT/US2018/026847
(87) International publication number: WO 2018/191238

(56) References cited:
- CEDRIC MATON ET AL: "Continuous Synthesis of Peralkylated Imidazoles and their Transformation into Ionic Liquids with Improved (Electro)Chemical Stabilities", CHEMPHYSCHEM - A EUROPEAN JOURNAL OF CHEMICAL PHYSICS & PHYSICALCHEMISTRY., vol. 13, no. 13, 22 June 2012 (2012-06-22) , pages 3146-3157, XP055479973, DE ISSN: 1439-4235, DOI: 10.1002/cphc.201200343
- KSHAMA SHETTY S ET AL: "Eco-friendly benzimidazolium based ionic liquid as a corrosion inhibitor for aluminum alloy composite in acidic media", JOURNAL OF MOLECULAR LIQUIDS, vol. 225, 25 November 2016 (2016-11-25), pages 426-438, XP029869326, ISSN: 0167-7322, DOI: 10.1016/J.MOLLIQ.2016.11.037
- B J Gour-Salin: "Hydrolysis rates of some acetylimidazole derivatives", Can. J . Chem., vol. 61 1 January 1983 (1983-01-01), pages 2059-2061, XP055479938, Retrieved from the Internet: URL:http://www.nrcresearchpress.com/doi/pd f/10.1139/v83-357
- CARL J. LOVELY ET AL: "Oxidative Rearrangement of Imidazoles with Dimethyldioxirane", ORGANIC LETTERS , 14(23), 6012-6015 CODEN: ORLEF7; ISSN: 1523-7052, vol. 6, no. 5, 1 March 2004 (2004-03-01), pages 735-738, XP055479936, ISSN: 1523-7060, DOI: 10.1021/ol036403w
- NICOLAS DUGUET ET AL: "Chiral relay in NHC-mediated asymmetric [beta]-lactam synthesis I; substituent effects in NHCs derived from (1R,2R)-cyclohexane-1,2-diamine", TETRAHEDRON ASYMMETRY, vol. 21, no. 5, 1 March 2010 (2010-03-01), pages 582-600, XP055479972, OXFORD, GB ISSN: 0957-4166, DOI: 10.1016/j.tetasy.2010.03.001
- KWANG-MING LEE ET AL: "N,N'-Dihexylbenzimidazolium salts. Anion-controlled bilayer structures via[pi]-[pi] dimer or C-H...[pi] catemer motifs", CRYSTENGCOMM, vol. 12, no. 12, 1 January 2010 (2010-01-01), pages 4347-4351, XP055479969, DOI: 10.1039/c0ce00105h

## Description

### FIELD

This disclosure relates to ionic liquids for use as coatings and adhesives that can be separated or removed from a surface to which they are applied by applying electromotive force to the compound or material.

### BACKGROUND

Certain electro-debonding compositions are known which may be used as an adhesive coating, such as the composition comprising commercial 1-ethyl-3-methylimidazolium bis(fluorosulfonyl)imide, sulfonylimide, functionalized ionic liquids, or imidazolium analogues used in power storage devices. However, the composition comprising 1-ethyl-3-methyl-imidazolium and/or bis(fluorosulfonyl)imide as coating material can be relatively corrosive to an aluminum surface.

Additionally most ionic liquids are strongly hydrophilic and hygroscopic. They dissolve in water well and absorb water readily from the ambient atmosphere. In applications where dry environment is required, such ionic liquids are not suitable to use. Ionic liquids comprising imidazolium cations with good thermal and electrochemical stability are disclosed in CEDRIC MATON ET AL: "Continuous Synthesis of Peralkylated Imidazoles and their Transformation into Ionic Liquids with Improved (Electro)Chemical Stabilities", CHEMPHYSCHEM - A EUROPEAN JOURNAL OF CHEMICAL PHYSICS & PHYSICALCHEMISTRY., vol. 13, no. 13, 22 June 2012, pages 3146-3157.

Thus, there is a need for a new hydrophobic ionic compound or composition that can be debonded from a surface without corrosiveness to the metallic substrates once coated.

### SUMMARY

According to a first aspect of the present invention there is provided an ionic compound comprising a 4,5,6,7-tetrahydro-benzoimidazolium cation, according to the Formula: 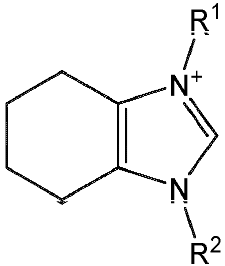 wherein R¹ and R² are independently optionally substituted C₃₋₇ alkyl or optionally substituted -(C₁₋₃ alkyl)-O-(C₁₋₃ alkyl) and wherein the cation is symmetrical.

Some embodiments include a composition comprising an ionic compound containing a 4,5,6,7-tetrahydro-benzoimidazolium cation, further comprising an anion according to the Formula:

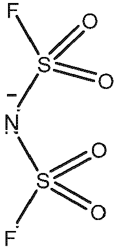

According to a second aspect of the present invention there is provided a device according to claim 13.

According to a third aspect of the present invention there is provided a method according to claim 14.

### BRIEF DESCRIPTION OF THE DRAWINGS

FIG. 1 is a schematic diagram of a device incorporating an embodiment of the use of an ionic compound or composition described herein.
FIG. 2 is a schematic diagram of a device incorporating an embodiment of the use of an ionic compound or composition described herein.
FIG. 3 is a schematic diagram of a device used in testing the adhesion properties of the ionic compounds described herein.
FIG. 4 is a plot of peeling strength density vs. time of an ionic compound or composition described herein tested in the device shown in FIG. 3.

### DETAILED DESCRIPTION

As used herein, when a compound or chemical structural is referred to as being "optionally substituted", it includes a feature that has no substituents (i.e. unsubstituted), or a feature that is "substituted," meaning that the feature has one or more substituents. The term "substituent" is broad, and includes a moiety that occupies a position normally occupied by one or more hydrogen atoms attached to a parent compound or structural feature. Any suitable substituent may be used, including substituents having a molecular weight of 15-50 g/mol, 15-100 g/m, or 15-200 g/mol, and substituents composed of atoms such as C, H, N, O, S, F, CI, Br, and/or I. In some embodiments, substituent groups are independently optionally substituted alkyl, alkenyl, alkoxy (e.g. -OCH₃, -OC₂H₅, -OC₃H₇, -OC₄H₉, etc.), alkylsulfones (e.g. -SCH₃, -SC₂H₅, -SC₃H₇, -SC₄H₉, etc.), -NR'R", -OH, -SH, -CN, -NO₂, or a halogen, wherein R' and R" are independently H or optionally substituted alkyl.

As used herein, the term "4,5,6,7-tetrahydro-benzoimidazolium cation" refers to the ring system having the following structure:

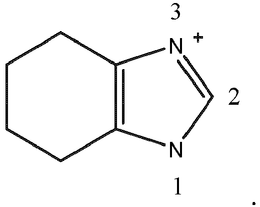

As used herein, the terms "bis(sulfonyl)imide and/or "sulfonyl imide" refers to a heteroatom moiety, for example, having the following structure:

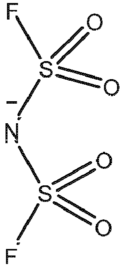

As used herein, the term "hydrophilic and/or hydrophilicity" refers to the preference of an ionic liquid (IL) to stay in water over an organic solvent. One useful test for determining relative hydrophobicity or hydrophilicity is to measure the partition ratio (or IL/DPM) of the test compound relative to an internal reference standard hydrophobic compound such as diphenylmethane (DPM) in an organic solvent. The partition ratio (or IL/DPM) is obtained by measuring the preference of the ionic liquid to stay in ethyl acetate over water relative to diphenyl methane as an internal reference standard. Typically, compounds having a partition ratio (IL/DPM) of less than about 0.5 would be considered hydrophilic.

As used herein, the term "hydrophobic and/or hydrophobicity" refers to the feature of an ionic liquid having preference of staying in an organic phase over water. Typically, compounds having a partition ratio (or IL/DPM) of greater than about 0.5 would be hydrophobic.

Some embodiments of the disclosure include an ionic liquid compound comprising an optionally substituted 4,5,6,7-tetrahydro-benzoimidazolium cation according to Formula 1: 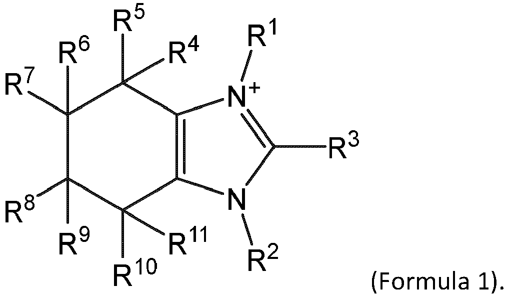 R¹ and R² are independently optionally substituted C₃₋₇ alkyl, or optionally substituted -(C₁₋₃ alkyl)-O-(C₁₋₃ alkyl); R³ is a substituent such as H or C₁₋₃ alkyl; and R⁴, R⁵, R⁶, R⁷, R⁸, R⁹, R¹⁰, or R¹¹ are independently any substituents such as the substituents as described below.

With respect of Formula 1, in some embodiments, R³, R⁴, R⁵, R⁶, R⁷, R⁸, R⁹, R¹⁰ and R¹¹ are all H, and the ionic liquid compound comprises a 4,5,6,7-tetrahydro-benzoimidazolium cation according to Formula 2: 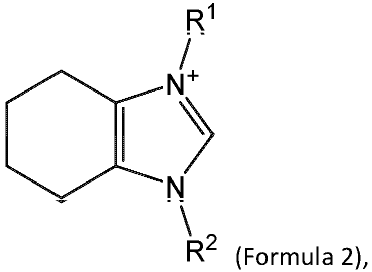 wherein, R¹ and R² are independently optionally substituted C₃₋₇ alkyl, or optionally substituted C₁₋₃ alkoxyalkyl; and wherein the cation is symmetrical.

With respect to any relevant structural representation, such as Formula 1 or Formula 2, in some embodiments, R¹ is: optionally substituted C₃₋₇ alkyl, such as optionally substituted C₃ alkyl (e.g. propyl), C₄ alkyl (e.g. butyl), C₅ alkyl (e.g. pentyl), C₆ alkyl (e.g. hexyl), or C₇ alkyl (e.g. heptyl). In some embodiments, R¹ is optionally substituted-(C₁₋₃ alkyl)-O-(C₁₋₃ alkyl), e.g. -(C₂ alkyl)-O-(C₁₋₃ alkyl) or -(C₂ alkyl)-O-(C₁₋₃ alkyl). In some embodiments, R¹ is isobutyl. In some embodiments, R¹ is neopentyl. In some embodiments, R¹ is n-hexyl. In some embodiments, R¹ is 2-ethoxyethyl.

With respect to any relevant structural representation, such as Formula 1 or Formula 2, in some embodiments, R² is: optionally substituted C₃₋₇ alkyl, such as optionally substituted C₃ alkyl (e.g. propyl), C₄ alkyl (e.g. butyl), C₅ alkyl (e.g. pentyl), C₆ alkyl (e.g. hexyl), or C₇ alkyl (e.g. heptyl). In some embodiments, R² is optionally substituted-(C₁₋₃ alkyl)-O-(C₁₋₃ alkyl), e.g. -(C₂ alkyl)-O-(C₁₋₃ alkyl) or -(C₂ alkyl)-O-(C₁₋₃ alkyl). In some embodiments, R² is isobutyl. In some embodiments, R² is neopentyl. In some embodiments, R² is n-hexyl. In some embodiments, R² is 2-ethoxyethyl.

In some embodiments, both R¹ and R² are n-hexyl. In some embodiments, both R¹ and R² are isobutyl.

In some embodiments, both R¹ and R² of Formula 1 or 2 can be hydrophilic functional group. In some embodiments, R¹ and R² can be hydrophilic functional group. In some embodiments, the hydrophilic functional group can comprise oxygen. In some embodiments, the hydrophilic functional group can be ether, hydroxyl, alkoxyl, or ester group.

In some embodiments, R¹ and R² of Formula 1 or 2 can be hydrophobic functional group. In some embodiments, R¹ and R² can be a hydrophobic functional group. In some embodiments, the hydrophobic functional group can comprise an optionally substituted alkyl group. In some embodiments, the optionally substituted alkyl group can comprise propyl, butyl, pentyl, hexyl, or hepatyl group.

The ionic compound of Formula 1 or 2, comprising a 4,5,6,7-tetrahydro-benzoimidazolium cation is symmetrical.

In some embodiments, R¹ and R² of Formula 1 or 2 are:

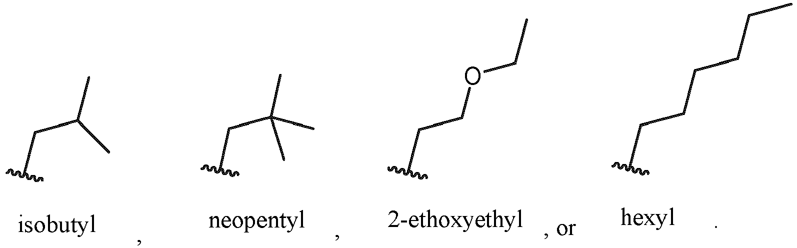

In some embodiments, 4,5,6,7-tetrahydro-benzoimidazolium cation of Formula 1 or 2 is:

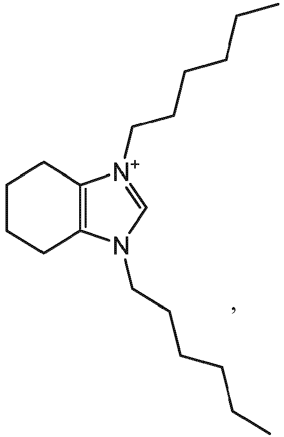

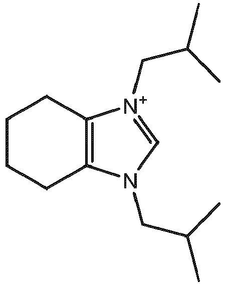

With respect to any relevant structural representation, such as Formula 1, in some embodiments, R³ is H, CH₃, C₂H₅, C₃H₇. In some embodiments, R³ is H.

With respect to any relevant structural representation, such as Formula 1, in some embodiments, R⁴ is H, CH₃, C₂H₅, C₃H₇, CH₃O (e.g. -OCH₃ or -CH₂OH), C₂H₅O, or C₃H₇O. In some embodiments, R⁴ is H.

With respect to any relevant structural representation, such as Formula 1, in some embodiments, R⁵ is H.

With respect to any relevant structural representation, such as Formula 1, in some embodiments, R⁶ is H, CH₃, C₂H₅, C₃H₇, CH₃O (e.g. -OCH₃ or -CH₂OH), C₂H₅O, or C₃H₇O. In some embodiments, R⁶ is H.

With respect to any relevant structural representation, such as Formula 1, in some embodiments, R⁷ is H.

With respect to any relevant structural representation, such as Formula 1, in some embodiments, R⁸ is H, CH₃, C₂H₅, C₃H₇, CH₃O (e.g. -OCH₃ or -CH₂OH), C₂H₅O, or C₃H₇O. In some embodiments, R⁸ is H.

With respect to any relevant structural representation, such as Formula 1, in some embodiments, R⁹ is H.

With respect to any relevant structural representation, such as Formula 1, in some embodiments, R¹⁰ is H, CH₃, C₂H₅, C₃H₇, CH₃O (e.g. -OCH₃ or -CH₂OH), C₂H₅O, or C₃H₇O. In some embodiments, R¹⁰ is H.

With respect to any relevant structural representation, such as Formula 1, in some embodiments, R¹¹ is H.

In some embodiments, the ionic composition can further comprise a bis(sulfonyl)imide anion, such as an anion with a structure shown in Formula 3:

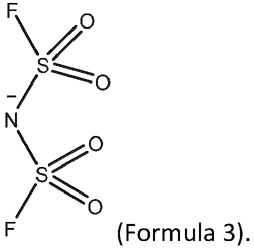

A selectively adhesive material comprising the ionic composition described above can bind a first electro-conducting surface and a second electro-conducting surface together, wherein an application of electromotive force to the electro-conductive surfaces reduces the adhesion of the adhesive material. Some of the selectively adhering adhesive compositions comprise a 4,5,6,7-tetrahydro-benzoimidazolium cation described above. The selectively adhesive composition, containing a 4,5,6,7-tetrahydro-benzoimidazolium cation, can further comprising a sulfonylimide anion, such as bis(fluorosulfonyl)imide anion described above.

Some of the selectively adhering adhesive described herein can further comprise a polymer to form a composite adhesive. The glass transition temperature of the polymer can be below about 0 °C. The polymer may be a polymer described in JP 2015-204998 and/or in JP 2015-204996. The polymer can be an acrylic polymer. The acrylic polymer can contain a monomer unit derived from a C₁₋₁₄ alkyl (or alkoxy) group containing alkyl (meth)acrylate ester, such as butyl (meth)acrylate. The monomer unit comprises a carboxyl group

Some embodiments include a device comprising any of the aforementioned ionic compounds or compositions. A suitable example of such a device can be as that described in JP 2015-204996 and/or in JP20204997.

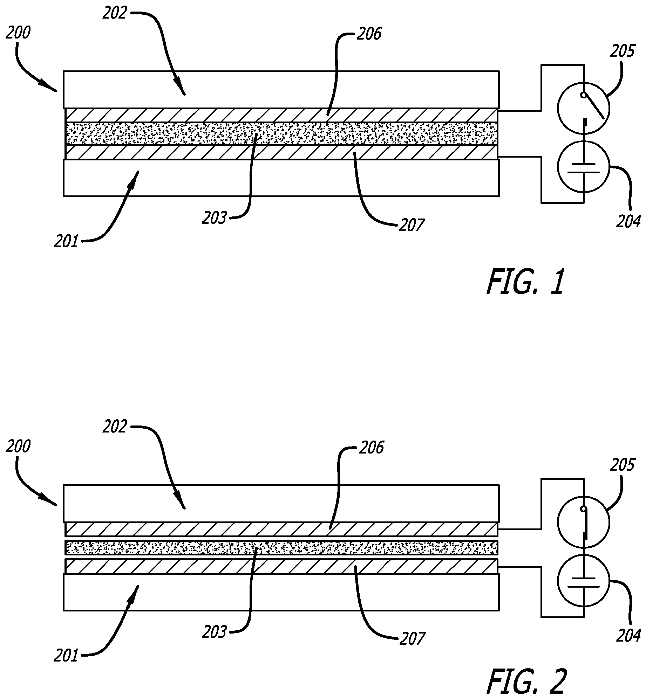

FIGS. 1 and 2 show some possible embodiments such as **200** of a composition described herein binding and connecting a first electro-conducting surface and a second electro-conducting surface together. As shown in FIGS. 1 and 2, adhesive **203,** incorporating the ionic compounds or compositions described herein, can be an adhesive layer or a coating disposed between first electro-conducting substrate **206** and second electro-conducting substrate **207.** First electro-conducting substrate **206** and second electro-conducting substrate **207** can disposed upon two non-metal (non-electro-conducting) substrates or layers, **201** and **202** respectively. The first and second electro-conducting substrates can be in electrical communication with a power supply **204,** e.g., a direct current power supply, to complete a closeable electrical circuit with an intervening switch **205.** When the switch **205** is closed, either or both of the two non-metal adherents **201** and **202** can separate the adhesive **203** from the either or both of the first and second electro-conducting substrates (separating metal and coating/adhesive at interfaces).

Any suitable electromotive force may be used to reduce the adhesion of the composition described herein so that the composition may be separated from one or two surfaces to which the composition is adhered. In some embodiments, the DC voltage can be about 3-100 V, about 3-5 V, about 5-10 V, about 10-20 V, about 20-30 V, about 30-50 V, about 50-70 V, about 60-80 V, about 80-100 V, about 10 V, or any DC voltage in a range bounded by any of these values.

Typically, when the composition is intended to be separated from one or more surfaces, the electromotive force that reduces the adhesive force between the composition and the surface can be smaller than the actual electromotive force applied to the composition. A DC voltage of about 3-90 V, about 3-5 V, about 5-10 V, about 10-20 V, about 20-30 V, about 30-50 V, about 50-70 V, about 60-80 V, about 80-90 V, about 10 V, or any DC voltage in a range bounded by any of these values, may reduce the adhesive force between the composition and the surface.

The electro-conductive substrate can comprise an electro-conducting carbonaceous material. The electro-conductive substrate can comprise an electroconducting metal. A conductive metal layer can comprise any electro-conducting metal, for example aluminum. The conductive metal layer may comprise a conventional material, such as a metal, mixed metal, alloy, metal oxide, and/or composite metal, or a conductive polymer. Examples of suitable metals include the Group 1 metals, the metals in Groups 4, 5, 6, and the Group 8-10 transition metals in the Periodical Table. Examples of suitable metals include stainless steel, Al, Ag, Mg, Ca, Cu, Mg/Ag, LiF/Al, CsF, and/or CsF/Al, and/or alloys thereof. The electro-conducting layers can have a thickness of about 1 nm to about 1000 µm. The electro-conducting layer has a thickness of about 20 nm to about 200 µm, about 20-200 nm, about 20-50 nm, about 50-100 nm, about 100-200 nm, about 200-500 nm, about 500-1000 nm, about 1-100 µm, about 100-200 µm, about 200-500 µm, about 500-100 µm, about 40-60 nm, or about 50 nm, or any thickness in a range bound by any of these values.

The selectively adherent adhesive comprising the ionic liquid compound described herein can have a reduced corrosive effect on the electro-conducting layer coated with such an adhesive. The selectively adherent adhesive comprising an ionic compound described herein can be used to coat on an electro-conductive layer to reduce the acidity of the environment immediately adjacent to the electro-conductive layer. Suitable methods to assess the corrosive effect of the adhesive coated or deposited on the electro-conducting materials can be the methods described in ASTM G69-12 (Standard Test Method for Measurement of Corrosion Potentials of Aluminum Alloys).

The selectively adherent adhesive composition comprising an ionic compound described herein can be used to coat or deposit on a substrate. The adhesive composition can be in a form of a layer or a sheet. The thickness of the layer or the sheet can be about 5 µm to about 100 µm, 5 µm to about 150 µm, about 5 µm to about 50 µm, about 5 µm to about 10 µm; about 10 µm to about 50 µm, about 50 µm to about 100 µm, or about 100 µm to about 150 µm, or any thickness in a range bounded by any of these values.

The selectively adherent adhesive composition comprising an ionic compound described herein, can further comprise an acrylic polymer, which can be used to coat or deposit on a substrate to form a composite composition. This composite composition can be in a form of a layer or a sheet. The thickness of the layer or the sheet comprising the composite composition can be about 50 µm to about 100 µm, 50 µm to about 150 µm, about 50 µm to about 200 µm, about 50 µm to about 100 µm, or about 100 µm to about 150 µm, or any thickness in a range bounded by any of these values.

The selectively adherent adhesive comprising the ionic compounds described herein can be chemically stable on an electrically conductive electrode or electro-conducting materials. The electrically conductive electrode can comprise an aluminum, stainless steel, and/or combinations or mixtures thereof. Chemical stability is defined as lack of (or minimal presence of) undesired reactions between a metal anode and the selectively adherent adhesive. Undesired reactions may include, for example, corrosive degradation of the metal electrode, dissolution of the metal in the selectively adherent adhesive, and/or pitting of the metal electrode. The aforementioned ionic composition deposited on, or in contact with the electrically conductive metal electrode may result in a reduced or absence of corrosive degradation mentioned above. Direct contact of the neat ionic compound upon the electrically conductive metal electrode may show any corrosive degradation mentioned above for a period of at least (or greater than) about 15 minutes, at least about 30 minutes, at least about 1 hour, at least about 3 hours, at least about 5 hours, at least about 7 hours, at least about 24 hours, at least about 50 hours, at least about 70 hours, at least about 100 hours, at least about 125 hours, at least about 200 hours, at least about 300 hours, or at least about 450 hours, or any time period in a range bounded by any of these values. Direct contact of the neat ionic composition upon the electrically conductive electrode may minimize and/or prevent corrosive degradation for a period of time as described above. In some embodiments, direct contact of the neat ionic composition upon the electrically conductive electrode may minimize and/or prevent corrosive degradation for a period of time as described above even under a high temperature of 85 °C, and high relative humidity of 85 %. No corrosive degradation is observed, as it lacks total penetration of an electro-conducting 50 nm-thick sheet of aluminum foil with the above described time frames, and/or environmental conditions.

The selectively adhesive compositions described herein can be formulated to minimize corrosion of the above-described electro-conducting substrates coated with such compositions, under conditions of high humidity and high temperature for prolonged time period. In particular, the adhesive composition can be capable of maintaining two such electro-conducting substrates in fixed relation to each other during and after being exposed to 85 °C and 85% relative humidity for a period of time described above.

The hydrophobicity of the ionic materials described herein can be determined by measuring the partition of the ionic materials between aqueous and organic phases. A suitable system utilizing water/ethyl acetate can be used to measure hydrophilic-hydrophobic behavior of ionic liquids. Diphenylmethane (DPM) can be used as an internal standard as a hydrophobic compound (assuming 100% hydrophobic). Thus, a solution of ionic liquid (0.5 mmol) and diphenylmethane (0.5 mmol) in ethyl acetate (100 mL) can be shaken with water (100 mL). After phase separation, ethyl acetate in the organic phase can be removed by evaporation under reduced pressure to leave a mixture of ionic liquid and DPM. The molar ratio of these two chemicals can be measured by Nuclear Magnetic Resonance (NMR) spectroscopy. The value obtained by this test, or the ratio: (moles ionic liquid/moles diphenylmethane), is referred to herein as "partition ratio" or "IL/DPM." If IL/DPM is more than 0.5, suggesting that the ionic liquid prefers to stay in ethyl acetate phase over water phase, then the ionic liquid is considered hydrophobic. Likewise, If an ionic liquid prefers to stay in water phase over ethyl acetate phase (IL/DPM < 0.5), the ionic liquid is considered hydrophilic. IL/DPM can be about 0.77 indicating that the ionic liquid is moderate hydrophobic. IL/DPM can be as high as 1.0, which is considered strongly hydrophobic. Some ionic compounds are hydrophobic having IL/DPM greater than 0.5 and up to 1.0.

The hydrophobicity of the materials can be determined by measuring the Water Contact Angle (WCA). Higher water contact angles are indicative of increased hydrophobicity. In other words, the greater the WCA, the less spread out a drop of water is on a surface. Increased water contact angles are indicative of less surface wetting and thus increased hydrophobicity. However, the hydrophobicity of a solution can also be measured by dropping it on an oil-compatible substrate, such as a polymer (e.g., PMMA). In this case, the relationship is reversed, and lower Solution Contact Angle (SCA) can be indicative of higher hydrophobicity. In some embodiments, the compounds described herein can have a SCA of less than about 35°, about 30-35 degree, about 25-30 degree, about 20-25 degree, about 15-20 degree, about 10-15 degree, about 10-20 degree, about 30°, about 25°, about 20°, about 18°, or about 16°.

In some embodiments, the hydrophobicity of the ionic materials can be observed as being insoluble in water, but soluble in a water-miscible organic solvent.

### EXAMPLES

Described herein are ionic compositions and elements that can reduce the deterioration and/or corrosion of the conductive metal layers described herein. These benefits are further shown by the following examples, which are intended to be illustrative of the embodiments of the disclosure, but are not intended to limit the scope or underlying principles in any way.

The abbreviation for some of the compounds or solvents used in the following examples are described below:
KOH: potassium hydroxide
DCM: dichloromethane
DMSO: dimethyl sulfoxide
KFSI: potassium fluorosilicate
THF: tetrahydrofuran

### Example 1: 1,3-Diethyl-4,5,6,7-tetrahydro-1H-benzo[d]imidazol-3-ium bis(fluorosulfonyl)amide (S1) - not within the scope of the invention

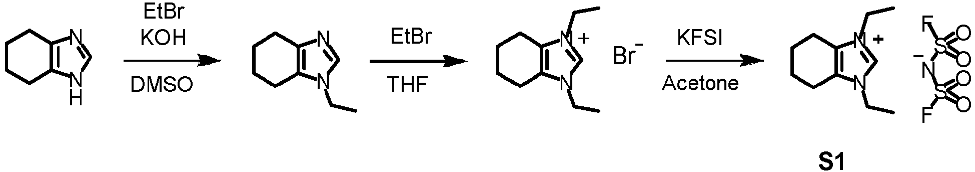

A mixture of 4,5,6,7-tetrahydro-1*H*-benzo[*d*]imidazole (3.60 g, 28.5 mmol), 90% KOH (2.49 g, 40 mmol) and DMSO (60 mL) was stirred under argon at room temperature for 16 h. Ethyl bromide (2.6 mL, 35 mmol) was added dropwise while the reaction mixture was cooled externally to keep its temperature in the range of 15-20 °C. Stirring at room temperature was then continued for 6 h. The reaction mixture was poured into ice/water (500 mL), treated with 5 N NaOH (100 mL) and extracted with DCM (2 x 200 mL). The extract was washed with water (200 mL), dried over Na₂CO₃, and the solvent was removed under reduced pressure to give 1-ethyl-4,5,6,7-tetrahydro-1*H*-benzo[*d*]imidazole (2.17 g, 49% yield).

A solution of 1-ethyl-4,5,6,7-tetrahydro-1*H*-benzo[*d*]imidazole (2.00 g, 13.3 mmol) and ethyl bromide (1.00 mL) in dry THF (20 mL) was stirred under argon and heated at 60 °C for 30 h. Two layers were formed. Upon standing at room temperature overnight, the bottom layer solidified. The solid was separated and recrystallized from acetone with cooling in dry ice to give 1,3-diethyl-4,5,6,7-tetrahydro-1*H*-benzo[*d*]imidazol-3-ium bromide (1.95 g, 56% yield).

A mixture of 1,3-diethyl-4,5,6,7-tetrahydro-1*H*-benzo[*d*]imidazol-3-ium bromide (1.87 g, 7.21 mmol), potassium salt of bis(fluorosulfonyl)imide (KFSI) (1.58 g, 7.21 mmol) and acetone (20 mL) was stirred under argon and heated at 50 °C for 3 h. After cooling to room temperature, the solid was filtered off, and the solvent was removed under reduced pressure. A solution of the residue in anhydrous THF (50 mL) was kept at room temperature overnight, then filtered, and concentrated under reduced pressure to give 1,3-diethyl-4,5,6,7-tetrahydro-1*H*-benzo[*d*]imidazol-3-ium bis(fluorosulfonyl)amide, S1 (2.50 g, 96% yield). ¹H NMR (DMSO-d₆): δ 9.10 (s, 1H), 4.10 (q, J = 7.5 Hz, 4H), 2.62 (bs, 4H), 1.79 (bs, 4H), 1.39 (t, J = 7.5 Hz, 6H).

### Example 2: 1,3-Dihexyl-4,5,6,7-tetrahydro-1H-benzo[d]imidazol-3-ium bis(fluorosulfonyl)amide (S2)

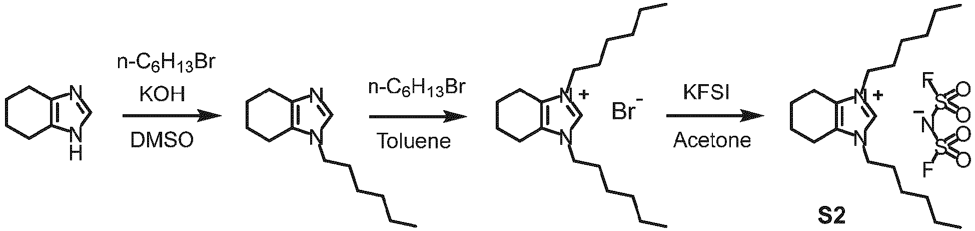

A mixture of 4,5,6,7-tetrahydro-1*H*-benzo[*d*]imidazole (5.00 g, 40.9 mmol), 90% KOH (3.74 g, 60 mmol) and DMSO (70 mL) was stirred under argon and heated at 40 °C for 2 h. After cooling to 10 °C, 1-bromohexane (6.3 mL, 45 mmol) was added in one portion, and the resulting mixture was stirred and heated at 40 °C for 4 days. The reaction mixture was poured into ice/water (600 mL), treated with 5 N NaOH

(100 mL) and extracted with DCM (2 x 400 mL). The extract was washed with water (300 mL), dried over Na₂SO₄, and the solvent was removed under reduced pressure to give a residue. Column chromatography of the residue (silica gel, ethyl acetate/methanol, 97:3) afforded pure 1-hexyl-4,5,6,7-tetrahydro-1*H-*benzo[*d*]imidazole (6.54 g, 77% yield).

A solution of 1-hexyl-4,5,6,7-tetrahydro-1*H*-benzo[*d*]imidazole (3.45 g, 16.7 mmol) and 1-bromohexane (3.5 mL, 25.0 mmol) in dry toluene (50 mL) was stirred under argon and heated at 100 °C for 24 h. The volatiles were removed under reduced pressure. The residue was triturated with ethyl ether (50 mL). The oily bottom phase was separated and dried in a vacuum oven to give 1,3-dihexyl-4,5,6,7-tetrahydro-1*H*-benzo[*d*]imidazol-3-ium bromide (5.99 g, 96% yield).

A mixture of 1,3-dihexyl-4,5,6,7-tetrahydro-1H-benzo[d]imidazol-3-ium bromide (2.89 g, 7.78 mmol), KFSI (1.705 g, 7.78 mmol) and acetone (50 mL) was stirred under argon and heated at 50 °C for 2 h. After cooling to room temperature, the solid was filtered off, and the solvent was removed under reduced pressure to give a residue. A solution of the residue in anhydrous THF (100 mL) was kept at room temperature overnight, then filtered, and concentrated under reduced pressure. A solution of the crude product in ethyl acetate (100 mL) was washed with water (2 x 100 mL), dried over Na₂SO₄, and the solvent was removed under reduced pressure to give 1,3-dihexyl-4,5,6,7-tetrahydro-1*H*-benzo[*d*]imidazol-3-ium bis(fluorosulfonyl)amide, S2 (3.50 g, 95% yield). ¹H NMR (DMSO-d₆): δ 9.02 (s, 1H), 4.05 (t, J = 7.5 Hz, 4H), 2.61 (bs, 4H), 1.80 (bs, 4H), 1.74 (quintet, J = 6.5 Hz, 4H), 1.28 (bs, 12H), 0.87 (t, J = 7.0 Hz, 6H) ppm.

### Reference Example 3: 3-(2-Ethoxyethyl)-1-hexyl-4,5,6,7-tetrahydro-1H-benzo[d]imidazol-3-ium bis(fluorosulfonyl)amide (S3) - not within the scope of the invention

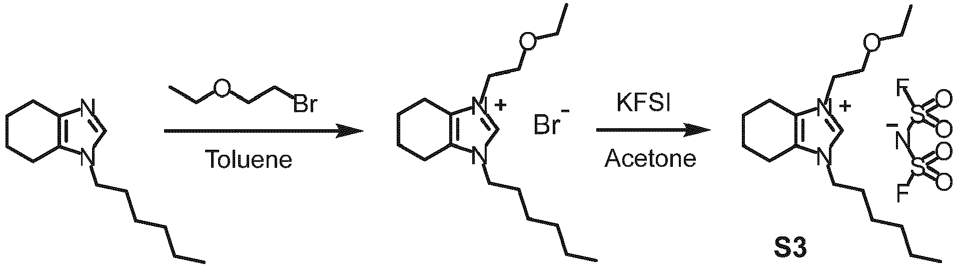

A solution of 1-hexyl-4,5,6,7-tetrahydro-1*H*-benzo[*d*]imidazole (3.10 g, 15.0 mmol) and 2-bromoethyl ethyl ether (2.8 mL, 25.0 mmol)) in dry toluene (50 mL) was stirred under argon and heated at 100 °C for 24 h. The volatiles were removed under reduced pressure. The residue was triturated with ethyl ether (50 mL). The oily bottom phase was separated and dried in a vacuum oven to give 3-(2-ethoxyethyl)-1-hexyl-4,5,6,7-tetrahydro-1*H*-benzo[*d*]imidazol-3-ium bromide (5.18 g, 96% yield).

A mixture of 3-(2-ethoxyethyl)-1-hexyl-4,5,6,7-tetrahydro-1*H-*benzo[*d*]imidazol-3-ium bromide (5.16 g, 14.4 mmol), KFSI (3.15 g, 14.4 mmol) and acetone (50 mL) was stirred under argon and heated at 50 °C for 2 h. After cooling to room temperature, the solid was filtered off, and the solvent was removed under reduced pressure to give a residue. A solution of the residue in anhydrous THF (100 mL) was kept at room temperature overnight, then filtered and concentrated under reduced pressure to provide a crude product. A solution of the crude product in ethyl ether/ethyl acetate (50 mL/50 mL) was washed with water (50 mL), dried over Na₂SO₄, and the solvent was removed under reduced pressure to give 3-(2-ethoxyethyl)-1-hexyl 4,5,6,7-tetrahydro-1*H*-benzo[*d*]imidazol-3-ium bis(fluorosulfonyl)amide, S3 (5.37 g, 81% yield). ¹H NMR (DMSO-d₆): δ 8.96 (s, 1H), 4.25 (t, J = 5.0 Hz, 2H), 4.08 (t, J = 7.5 Hz, 2H), 3.67 (t, J = 5.0 Hz, 2H), 3.44 (q, J = 7.0 Hz, 2H), 2.62 (bs, 4H), 1.79 (bs, 4H), 1.72 (quintet, J = 6.5 Hz, 2H), 1.28 (bs, 6H), 1.07 (t, J = 7.0 Hz, 3H), 0.87 (t, J = 7.0 Hz, 3H) ppm.

### Reference Example 4: 1-Hexyl-3-neopentyl-4,5,6,7-tetrahydro-1H-benzo[d]imidazol-3-ium bis(fluorosulfonyl)amide (S4) - not within the scope of the invention

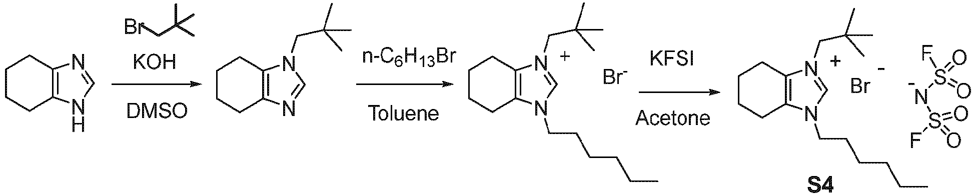

A mixture of 4,5,6,7-tetrahydro-1*H*-benzo[*d*]imidazole (5.00 g, 40.9 mmol), 90% KOH (3.74 g, 60 mmol) and DMSO (70 mL) was stirred under argon and heated at 40 °C for 2 h. After cooling to 10 °C, neopentyl bromide (5.5 mL, 44 mmol) was added in one portion, and the resulting mixture was stirred and heated at 40 °C for 4 days. The reaction mixture was poured into ice/water (800 mL), treated with 5 N NaOH (200 mL), and extracted with DCM (2 x 400 mL). The extract was washed with water (200 mL), dried over Na₂SO₄, and the solvent was removed under reduced pressure to give a residue. Column chromatography of the residue (silica gel, ethyl acetate/methanol, 95:5) afforded pure 1-neopentyl-4,5,6,7-tetrahydro-1*H-*benzo[*d*]imidazole (4.72 g, 60% yield).

A solution of 1-neopentyl-4,5,6,7-tetrahydro-1*H*-benzo[*d*]imidazole (4.60 g, 23.9 mmol) and 1-bromohexane (8.0 mL, 57.0 mmol) in dry toluene (75 mL) was stirred under argon and heated at 100 °C for 24 h. The volatiles were removed under reduced pressure. The residue was triturated with ethyl ether (50 mL) and separated by decantation. To remove its dark color, the product was dissolved in methanol (100 mL), treated with activated carbon and stirred overnight. The carbon was filtered off using a pad of Celite, and the solvent was removed under reduced pressure to give pure 1-hexyl-3-neopentyl-4,5,6,7-tetrahydro-1*H*-benzo[*d*]imidazol-3-ium bromide (5.00 g, 58% yield).

A mixture of 1-hexyl-3-neopentyl-4,5,6,7-tetrahydro-1*H*-benzo[*d*]imidazol-3-ium bromide (5.00 g, 14.0 mmol), KFSI (3.07 g, 14.0 mmol) and acetone (60 mL) was stirred under argon and heated at 60 °C for 2 h. After cooling to room temperature, the solid was filtered off, and the solvent was removed under reduced pressure. A solution of the residue in anhydrous THF (100 mL) was kept at room temperature overnight, then filtered and concentrated under reduced pressure to give 1-hexyl-3-neopentyl-4,5,6,7-tetrahydro-1*H*-benzo[*d*]imidazol-3-ium bis(fluorosulfonyl)amide, S4 (6.20 g, 97% yield). ¹H NMR (DMSO-d₆): δ 8.99 (s, 1H), 4.10 (t, J = 7.0 Hz, 2H), 3.90 (s, 2H), 2.61 (m, 4H), 1.78 (m, 4H), 1.74 (m, 2H), 1.26 (bs, 6H), 0.95 (s, 9H), 0.86 (t, J = 7.0 Hz, 3H) ppm.

### Example 5: 1,3-Diisobutyl-4,5,6,7-tetrahydro-1H-benzo[d]imidazol-3-ium bis(fluorosulfonyl)amide (S5)

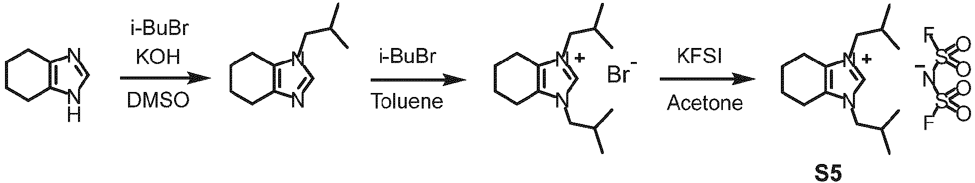

A mixture of 4,5,6,7-tetrahydro-1*H*-benzo[*d*]imidazole (6.00 g, 49.1 mmol), 90% KOH (4.35 g, 70 mmol) and DMSO (80 mL) was stirred under argon and heated at 50 °C for 1 h. After cooling to 10 °C, isobutyl bromide (5.7 mL, 52 mmol) was added portion-wise within 1 h while keeping temperature in the range of 10-15 °C, and the resulting mixture was then stirred at room temperature for 40 hours. The reaction mixture was poured into ice/water (400 mL), treated with 5 N NaOH (100 mL) and extracted with DCM (2 x 200 mL). The extract was washed with water (200 mL), dried over Na₂SO₄, and the solvent was removed under reduced pressure to give a residue. Column chromatography of the residue (silica gel, ethyl acetate/methanol, 95:5) afforded pure 1-isobutyl-4,5,6,7-tetrahydro-1*H*-benzo[*d*]imidazole (5.62 g, 64% yield).

A mixture of 1-isobutyl-4,5,6,7-tetrahydro-1*H*-benzo[*d*]imidazole (5.50 g, 30.8 mmol), isobutyl bromide (5.4 mL, 50 mmol) and toluene (75 mL) was stirred under argon and heated at 100 °C for 16 h. TLC indicated that only about a half of the starting material reacted. Temperature was increased to 120 °C, and the reaction was continued for additional 20 h. The solvent was removed under reduced pressure. The residue was washed thoroughly with ethyl ether and dried in a vacuum oven at 80 °C to give 1,3-diisobutyl-4,5,6,7-tetrahydro-1*H*-benzo[*d*]imidazol-3-ium bromide (5.50 g, 57% yield).

A mixture of 1,3-diisobutyl-4,5,6,7-tetrahydro-1*H*-benzo[*d*]imidazol-3-ium bromide (4.80 g, 15.2 mmol), KFSI (3.29 g, 15.0 mmol) and acetone (50 mL) was stirred under argon and heated at 50 °C for 2 h. After cooling to room temperature, the solid was filtered off, and the solvent was removed under reduced pressure. A solution of the residue in ethyl acetate (150 mL) was washed with water (100 mL) adjusted pH of the aqueous layer to 8.0 with saturated sodium bicarbonate, dried over anhydrous sodium sulfate, and concentrated under reduced pressure to give a residue. The residue was triturated with a mixture of ethyl ether/ hexanes (1:1, 100 mL) to give crystalline 1,3-diisobutyl-4,5,6,7-tetrahydro-1*H*-benzo[*d*]imidazol-3-ium bis(fluorosulfonyl)amide, S5 (4.79 g, 76% yield). ¹H NMR (DMSO-d₆): δ 9.02 (s, 1H), 3.92 (d, J = 7.5 Hz, 4H), 2.61 (bs, 4H), 2.02 (m, 2H), 1.80 (bs, 4H), 0.89 (d, J = 6.5 Hz, 12H) ppm.

### Example 6: Measuring hydrophobicity of ionic liquids

A system containing water/ethyl acetate was designed to measure hydrophilic-hydrophobic behavior of ionic liquids. Diphenylmethane (DPM) is used as an internal standard. Thus, a solution of ionic liquid (0.5 mmol) and diphenylmethane (0.5 mmol) in ethyl acetate (100 mL) was shaken with water (100 mL). After phase separation, ethyl acetate in the organic layer was removed by evaporation under reduced pressure to leave a mixture of ionic liquid and DPM. The molar ratio of these two chemicals was then determined by NMR analysis. Assuming DPM represents to be 100% hydrophobic, the molar ratio of ionic liquid to DPM indicates how hydrophobic an ionic liquid is. For the purposes of this disclosure, the value obtained by this test will referred to as the "partition ratio," or "IL/DPM." If an ionic liquid (IL) prefers to stay in water over organic layer (such as ethyl acetate) (IL/DPM < 0.5), we call it hydrophilic. If an ionic liquid prefers to stay in organic layer (such as ethyl acetate) over aqueous layer, (IL/DPM > 0.5), we call it hydrophobic. The data for two ionic liquids (S1 and S2) and a reference compound CE-1 are given below.

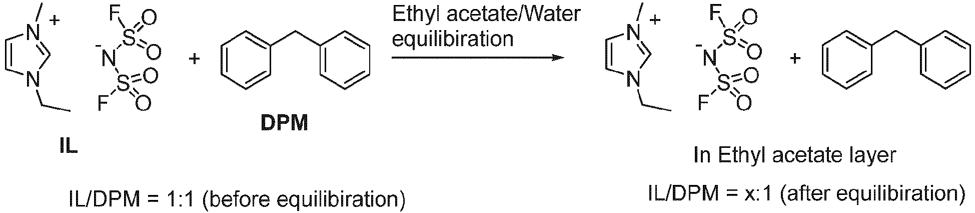

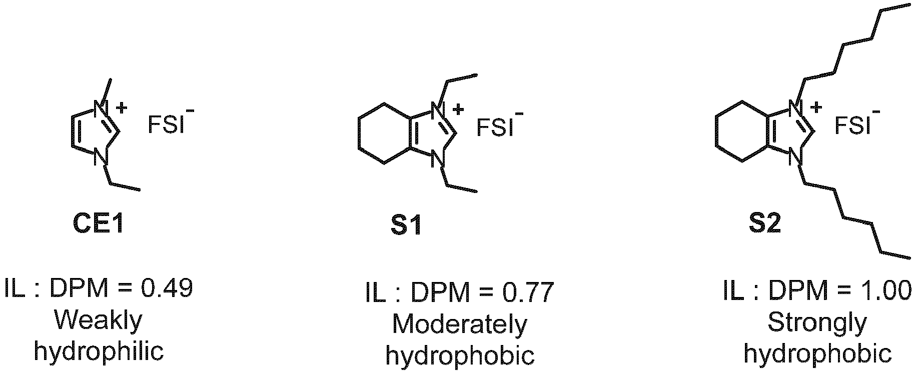

### Example 7: Preparation of polymer solution

n-Butyl acrylate (BA) (95 mass parts), acrylic acid (AA) (5 mass parts) and ethyl acetate (125 mass parts) were introduced into a flask (containing a stirring bar) attached to a condenser that was equipped with a nitrogen gas inlet. The mixture was stirred at room temperature while introducing the nitrogen gas for about 1 hour to remove oxygen from the reaction system. Azobisisobutyronitrile (AIBN) (0.2 mass parts) was added, which increased the temperature of the resulting mixture to about 63±2 °C, and the resulting mixture was stirred for about 5-6 hours for polymerization. After stopping heating, an acrylic polymer-containing solution was obtained with a concentration of about 30 wt%. The apparent molecular weight of the polymer (P1) was determined to be about 800,000, with a Tg (glass transition temperature) of about -50°C.

### Example 8: Preparation of adhesive sheet

First, an electrically debondable adhesive composition was obtained by mixing the polymer solution described above with 0.01 gram of an epoxy crosslinking agent, such as N,N,N',N'-tetraglycidyl-m-xylenediamine, per 100 gram of polymer solution, and one of the ionic liquid compounds (1.5 wt%) described above. The prepared compositions were then coated/deposited upon a surface treated PET separator (release liner) [MRF38, made by Mitsubishi Chemical Corp., Japan], forming an adhesive composite layer at a thickness of about 150 µm (microns). The coated film was then heat dried at 130 °C for about 3 minutes. A second PET separator (release liner) was then aligned over the exposed adhesive coating to obtain a layered sheet (PET separator/adhesive coating/PET separator) which was then aged/dried at 50 °C for about 20-24 hours and then stored under ambient conditions until needed.

### Example 9. Adhesive Ionic Composition Corrosive Test

Just prior to the application of the adhesive sheet to the nano-AI coated layer, the aforementioned release liner was removed. The adhesive sheet, as previously described above was applied to the metallic surface of the aluminum film (50 nm-thick aluminum coated PET film [Toray Advanced Film, Tokyo, Japan]).

The prepared film was placed in a Temperature & Humidity Benchtop chamber, set at 85°C and 85% Relative Humidity (ESPEC North America, [Hudsonville, MI, USA], Criterion Temperature & Humidity Benchtop Model BTL-433) and were checked at 70 hours. The interface between the adhesive and the aluminum foil was visually examined for an indication of corrosive degradation of the aluminum foil and/or dissolution of the metal in the selectively adherent adhesive and/or pitting of the aluminum foil. If any of the corrosiveness described herein was not visually observed, the sample was indicated as "No Corrosion". If any of the corrosiveness described herein was observed and was as extensive as (or close to) the corrosiveness visually observed for CE-1, the sample is indicated as "Excessive Corrosion". If very little of the corrosiveness described herein was visually observed and was significantly less corrosive than that was visually observed for CE-1, the sample was indicated as "Slight Corrosion". The results are summarized in Table 1, below.

**Table 1.**

| **No IL** | **CE-1** | **S1** | **S2** | **S3** | **S4** | **S5** |
|---|---|---|---|---|---|---|
| No Corrosion | Excessive Corrosion | Excessive Corrosion | No Corrosion | Slight Corrosion | Excessive Corrosion | No Corrosion |

### Example 10: Adhesion testing of adhesive compositions

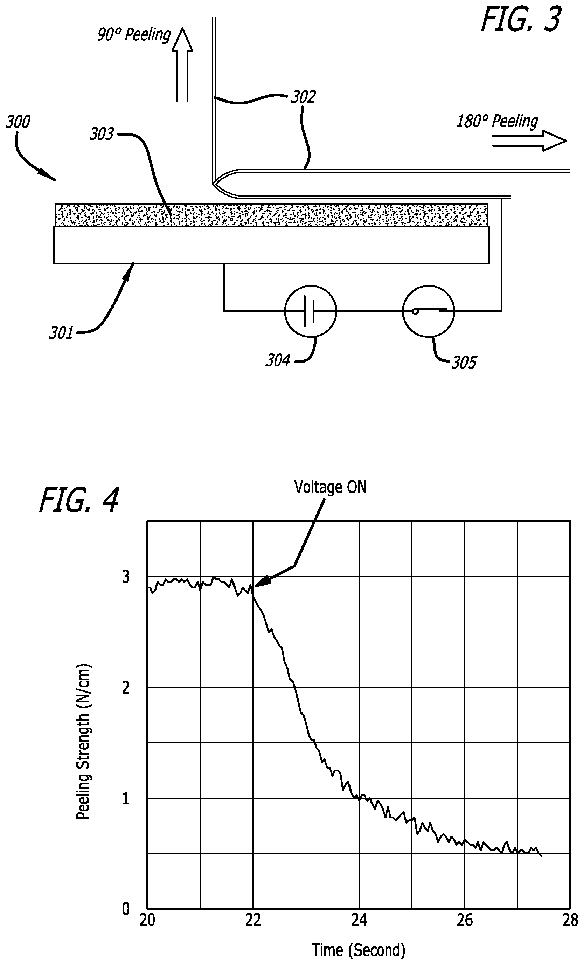

The testing for adhesion was done in the manner as described in JP 2015-228951 and/or JP 2015-204998, and also shown in FIG. 3.

As shown in FIG. 3, in the device **300,** the adhesive material **303** was coated upon a conductive substrate **301,** which is 25 mm wide and 100 mm long, and laminated by the application of rolling pressure, by 2 kg roller and roll press upon another flexible conductive layer **302** (such as aluminum foil and/or metalized plastic film such as PET), which is 10 mm to 25 mm wide and 100 mm longer than conductive substrate **301.**

The bonding/de-bonding tester (Mark-10, Copiague, New York, USA, model ESM303 motorized tension / compression stand) was equipped with a Mark-10 force gauge (Series 7-1000) and had lower and upper clamps. As shown in FIG. 3, the conductive substrate **301** as described in Example 2 was fixed onto the lower clamp and then electrically connected to the positive pole of a power supply **304** (Protek DC Power Supply 3006B). The top layer **302** was fixed to the upper clamp which is connected with the negative pole of the same power supply which was connected with a power on/off switch **305.** The power supply had an output range from 0 to 100 VDC.

In a dynamic test, the moving/peeling speed was set at 300 mm/min., and the voltage was applied a few seconds after the peeling or separation starts. The time and peeling strength readings from the force gauge are recorded by the data acquisition system (Mark-10 MESURgauge Plus). FIG. 4 shows the 180 degree peeling strength evolution over time when a 10 VDC was applied to the adhesive material that is doped with Compound S2 with a concentration of 5 wt.%.

In a static de-bonding test, the sample was fixed on to the tester and connected to the power supply in the same way. The peeling strength of the initial 180-degree peeling was measured at the same peeling speed of 300 mm/min. Then peeling was stopped. A DC voltage (10 VDC for example) was applied for some time (10 second for example). And then the peeling strength was measured at the same peeling speed of 300 mm/min. For the same adhesive sample from compound S2, the initial peeling strength is 3.0 N/cm, and the residual adhesion peeling strength is about 0.2 after applying 10 VDC for 10 second.

Unless otherwise indicated, all numbers expressing quantities of ingredients, properties such as molecular weight, reaction conditions, and so forth used in the specification and embodiments are to be understood as being modified in all instances by the term "about." Accordingly, unless indicated to the contrary, the numerical parameters set forth in the specification and attached embodiments are approximations that may vary depending upon the desired properties sought to be obtained. At the very least, and not as an attempt to limit the application of the doctrine of equivalents to the scope of the embodiments, each numerical parameter should at least be construed in light of the number of reported significant digits and by applying ordinary rounding techniques.

The terms "a," "an," "the" and similar referents used in the context of describing the invention (especially in the context of the following embodiments) are to be construed to cover both the singular and the plural, unless otherwise indicated herein or clearly contradicted by context. All methods described herein can be performed in any suitable order unless otherwise indicated herein or otherwise clearly contradicted by context. The use of any and all examples, or exemplary language (e.g., "such as") provided herein is intended merely to better illuminate the invention and does not pose a limitation on the scope of any embodiment. No language in the specification should be construed as indicating any non-claimed element essential o the practice of the invention.

## Claims

1. An ionic compound comprising a cation according to the formula: 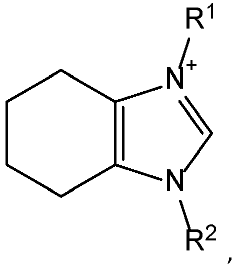 wherein R¹ and R² are optionally substituted C₃ alkyl, C₄ alkyl, C₅ alkyl, C₆ alkyl, or C₇ alkyl or optionally substituted -(C₁₋₃ alkyl)-O-(C₁₋₃ alkyl) and wherein the cation is symmetrical.

2. The ionic compound of claim 1, further comprising an anion according to the following formula:

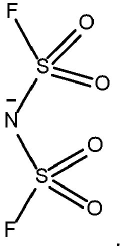

3. The ionic compound of claim 1 or 2, wherein R¹ and R² are isobutyl, neopentyl, n-hexyl or 2-ethoxyethyl.

4. The ionic compound of claim 1, 2, or 3, comprising:

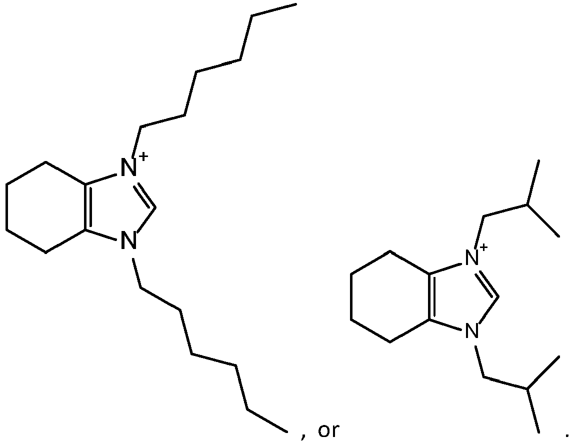

5. A composition, comprising an ionic compound of claim 1, 2, 3, or 4.

6. The composition of claim 5, further comprising an acrylic polymer to form a composite layer or a sheet.

7. The composition of claim 6, wherein the layer or the sheet has a thickness of 5 µm to 150 µm.

8. The composition of claim 6 or 7, wherein the layer or the sheet is formed on a conductive substrate.

9. The composition of claim 8, wherein the conductive substrate is a metal film, or a metalized plastic film, or a combination thereof.

10. The composition of claim 6, 7, 8, or 9, having an adhesive strength that reduces upon application of a 10-volt electric potential difference to the composition.

11. The composition of claim 6, 7, 8, 9, or 10, which is less corrosive than 1-ethyl-3-methyl-imidazolium bis(fluorosulfonyl)imide, as determined by the amount of contact time required to visually observe corrosion after directly applying the composition or 1-ethyl-3-methyl-imidazolium bis(fluorosulfonyl)imide to an aluminum foil.

12. The composition of claim 6, 7, 8, 9, 10, or 11, wherein the ionic compound has a partition ratio (IL/DPM) that is 0.5 to 1.

13. A device, comprising:
a first electroconducting substrate;
a second electroconducting substrate; and
a composition of claim 6, 7, 8, 9, 10, 11, or 12, positioned between the first electroconducting substrate and the second electroconducting substrates, and binding the first electroconducting substrate to the second electroconducting substrate;
wherein the composition has the property that an application of 10 volts electric potential difference between the first electroconducting substrate and the second electroconducting substrate reduces the adhesion of the composition.

14. A method comprising applying the composition of claim 6, 7, 8, 9, 10, 11, or 12, between a first electroconducting substrate and a second electroconducting substrate to adhere the first electroconducting substrate to the second electroconducting substrate, and applying an electrical current between the first electroconducting substrate and the second electroconducting substrate to separate the first electroconducting substrate or the second electroconducting substrate or both electroconducting substrates from the composition.

## Patentansprüche

1. Ionische Verbindung, umfassend ein Kation gemäß der Formel: 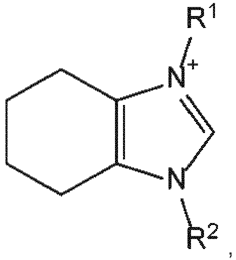 wobei R¹ und R² optional ein substituiertes C₃-Alkyl, C₄-Alkyl, C₅-Alkyl, C₆-Alkyl oder C₇-Alkyl oder optional ein substituiertes -(C₁₋₃-Alkyl)-O-(C₁₋₃-Alkyl) sind und wobei das Kation symmetrisch ist.

2. Ionische Verbindung nach Anspruch 1, ferner umfassend ein Anion gemäß der folgenden Formel:

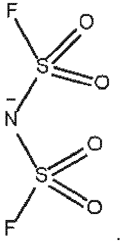

3. Ionische Verbindung nach Anspruch 1 oder 2, wobei R¹ und R² Isobutyl, Neopentyl, n-Hexyl oder 2-Ethoxyethyl sind.

4. Ionische Verbindung nach Anspruch 1, 2 oder 3, umfassend:

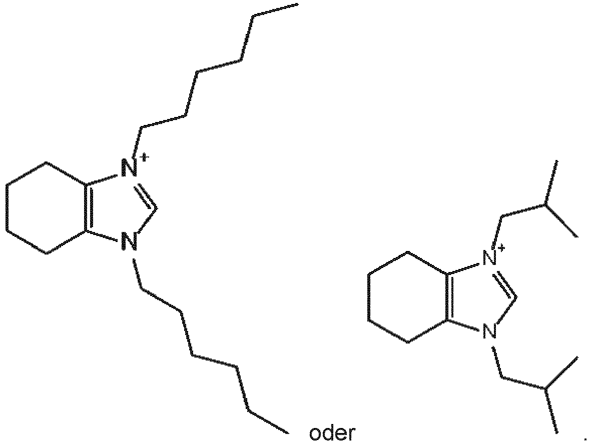

5. Zusammensetzung, umfassend eine ionische Verbindung nach Anspruch 1, 2, 3 oder 4.

6. Zusammensetzung nach Anspruch 5, ferner umfassend ein Acrylpolymer, um eine Verbundschicht oder eine Folie auszubilden.

7. Zusammensetzung nach Anspruch 6, wobei die Schicht oder die Folie eine Dicke von 5 µm bis 150 µm aufweist.

8. Zusammensetzung nach Anspruch 6 oder 7, wobei die Schicht oder die Folie auf einem leitenden Substrat ausgebildet ist.

9. Zusammensetzung nach Anspruch 8, wobei das leitende Substrat ein Metallfilm oder ein metallisierter Kunststofffilm oder eine Kombination davon ist.

10. Zusammensetzung nach Anspruch 6, 7, 8 oder 9, die eine Haftfestigkeit aufweist, die sich bei einem Anlegen eines elektrischen Potentials von 10 Volt an die Zusammensetzung verringert.

11. Zusammensetzung nach Anspruch 6, 7, 8, 9 oder 10, die weniger korrosiv als 1-Ethyl-3-methylimidazoliumbis(fluorsulfonyl)imid ist, wie durch die Menge an Kontaktzeit bestimmt ist, die erforderlich ist, um Korrosion nach direktem Aufbringen der Zusammensetzung oder von 1-Ethyl-3-methylimidazoliumbis(fluorsulfonyl)imid auf eine Aluminiumfolie visuell zu beobachten.

12. Zusammensetzung nach Anspruch 6, 7, 8, 9, 10 oder 11, wobei die ionische Verbindung einen Trenngrad (IL/DPM) aufweist, der 0,5 bis 1 beträgt.

13. Vorrichtung, umfassend:
ein erstes elektrisch leitendes Substrat;
ein zweites elektrisch leitendes Substrat; und
eine Zusammensetzung nach Anspruch 6, 7, 8, 9, 10, 11 oder 12, die zwischen dem ersten elektrisch leitenden Substrat und den zweiten elektrisch leitenden Substraten positioniert ist und
das erste elektrisch leitende Substrat an das zweite elektrisch leitende Substrat bindet;
wobei die Zusammensetzung die Eigenschaft aufweist, dass ein Anlegen eines elektrischen Potentials von 10 Volt zwischen dem ersten elektrisch leitenden Substrat und dem zweiten elektrisch leitenden Substrat die Haftung der Zusammensetzung verringert.

14. Verfahren, umfassend das Aufbringen der Zusammensetzung nach Anspruch 6, 7, 8, 9, 10, 11 oder 12 zwischen einem ersten elektrisch leitenden Substrat und einem zweiten elektrisch leitenden Substrat, um das erste elektrisch leitende Substrat an dem zweiten elektrisch leitenden Substrat haften zu lassen, und das Anlegen eines elektrischen Stroms zwischen dem ersten elektrisch leitenden Substrat und dem zweiten elektrisch leitenden Substrat, um das erste elektrisch leitende Substrat oder das zweite elektrisch leitende Substrat oder beide elektrisch leitenden Substrate von der Zusammensetzung zu trennen.

## Revendications

1. Composé ionique comprenant un cation selon la formule : 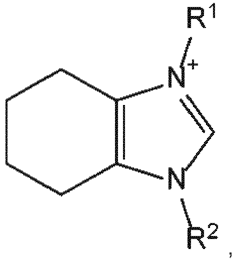 où R¹ et R² représentent un C₃ alkyle, C₄ alkyle, C₅ alkyle, C₆ alkyle, ou C₇ alkyle éventuellement substitués ou -(C₁₋₃ alkyl)-O-(C₁₋₃ alkyl) éventuellement substitué et où le cation est symétrique.

2. Composé ionique selon la revendication 1, comprenant en outre un anion selon la formule suivante :

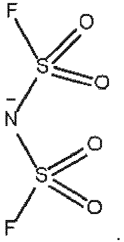

3. Composé ionique selon la revendication 1 ou 2, R¹ et R² représentant l'isobutyle, le néopentyle, le n-hexyle ou le 2-éthoxyéthyle.

4. Composé ionique selon la revendication 1, 2 ou 3, comprenant :

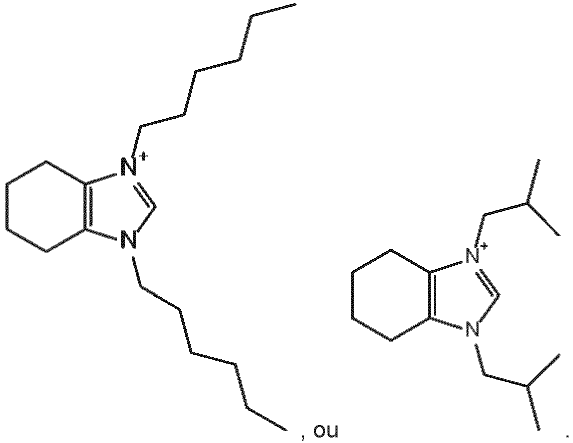

5. Composition, comprenant un composé ionique selon la revendication 1, 2, 3 ou 4.

6. Composition selon la revendication 5, comprenant en outre un polymère acrylique pour former une couche composite ou une feuille.

7. Composition selon la revendication 6, la couche ou la feuille ayant une épaisseur de 5 µm à 150 µm.

8. Composition selon la revendication 6 ou 7, la couche ou la feuille étant formée sur un substrat conducteur.

9. Composition selon la revendication 8, le substrat conducteur étant un film métallique, ou un film plastique métallisé, ou une combinaison de ceux-ci.

10. Composition selon la revendication 6, 7, 8 ou 9, ayant une force adhésive qui diminue lors de l'application d'une différence de potentiel électrique de 10 volts à la composition.

11. Composition selon la revendication 6, 7, 8, 9 ou 10, qui est moins corrosive que le 1-éthyl-3-méthyl-imidazolium bis(fluorosulfonyl)imide, tel que déterminé par la quantité de temps de contact nécessaire pour observer visuellement la corrosion après application directe de la composition ou du 1-éthyl-3-méthyl-imidazolium bis(fluorosulfonyl)imide sur une feuille d'aluminium.

12. Composition selon la revendication 6, 7, 8, 9, 10 ou 11, le composé ionique ayant un coefficient de partage (IL/DPM) qui est de 0,5 à 1.

13. Dispositif comprenant :
un premier substrat électroconducteur ;
un second substrat électroconducteur ; et
une composition selon la revendication 6, 7, 8, 9, 10, 11 ou 12, positionnée entre le premier substrat électroconducteur et les seconds substrats électroconducteurs, et la liaison du premier substrat électroconducteur au second substrat électroconducteur ;
la composition ayant la propriété selon laquelle une application d'une différence de potentiel électrique de 10 volts entre le premier substrat électroconducteur et le second substrat électroconducteur réduit l'adhérence de la composition.

14. Procédé comprenant l'application de la composition selon la revendication 6, 7, 8, 9, 10, 11 ou 12, entre un premier substrat électroconducteur et un second substrat électroconducteur pour faire adhérer le premier substrat électroconducteur au second substrat électroconducteur, et appliquer un courant électrique entre le premier substrat électroconducteur et le second substrat électroconducteur pour séparer le premier substrat électroconducteur ou le second substrat électroconducteur ou les deux substrats électroconducteurs de la composition.
